# EUROPÄISCHE PATENTSCHRIFT

(11) **EP 1 163 257 B1**
(45) Veröffentlichungstag und Bekanntmachung des Hinweises auf die Patenterteilung: **13.11.2002**
(21) Anmeldenummer: 00914141.7
(22) Anmeldetag: 16.03.2000
(51) Int. Cl.: C07J 7/00, C07J 1/00, C07J 13/00, C07J 9/00

(54) **VERFAHREN ZUR HERSTELLUNG VON 4,4- DIMETHYL -3BETA- HYDROXYPREGNA -8,14-DIEN-21- CARBONSÄUREESTERN UND ZWISCHENPRODUKTE IM VERFAHREN**
METHOD FOR PRODUCING 4,4-DIMETHYL-3BETA-HYDROXYPREGNA-8,14-DIENE-21-CARBOXYLIC ACID ESTERS AND INTERMEDIATE PRODUCTS OBTAINED BY SAID METHOD
PROCEDE PERMETTANT DE PRODUIRE DES ESTERS DE L'ACIDE 4,4-DIMETHYL-3BETA-HYDROXYPREGNA-8,14-DIENE-21-CARBOXYLIQUE ET PRODUITS INTERMEDIAIRES OBTENUS PAR LEDIT PROCEDE

(30) Priorität: 19.03.1999 DE 19914019
(43) Veröffentlichungstag der Anmeldung: 19.12.2001
(73) Patentinhaber: SCHERING AKTIENGESELLSCHAFT, 13342 Berlin (DE)
(72) Erfinder: GEISLER, Jens, D-13589 Berlin (DE); WINTER, Eric, D-13507 Berlin (DE)
(86) Internationale Anmeldenummer: EP0002323
(87) Internationale Veröffentlichungsnummer: WO00056758

(56) Entgegenhaltungen:
- WO-A-99/52930
- DOLLE R E ET AL: "SYNTHESIS OF ZYMOSTEROL, FECOSTEROL, AND RELATED BIOSYNTHETIC STEROL INTERMEDIATES 1,2" JOURNAL OF THE AMERICAN CHEMICAL SOCIETY,US,AMERICAN CHEMICAL SOCIETY, WASHINGTON, DC, Bd. 111, Nr. 1, 4. Januar 1989 (1989-01-04), Seiten 278-284, XP000611665 ISSN: 0002-7863
- R B WOODWARD ET AL: "The Synthesis of Lanosterol (Lanostadienol)" JOURNAL OF THE CHEMICAL SOCIETY,GB,CHEMICAL SOCIETY. LETCHWORTH, Bd. 3, Nr. 3, März 1957 (1957-03), Seiten 1131-1144-1144, XP002111485
- LETTRE ET AL: "Polyols derived from sterols and sterol derivatives. VI. Steroids containing structural units of ecdysone and the elatericins" JUSTUS LIEBIGS ANNALEN DER CHEMIE,DE,VERLAG CHEMIE GMBH. WEINHEIM, Bd. 758, Nr. 758, 1972, Seiten 89-110-110, XP002111484 ISSN: 0075-4617
- WENCKENS M ET AL: "SYNTHESIS OF MEIOSIS-ACTIVATING STEROLS CONTAINING FLUORINE" ACTA CHEMICA SCANDINAVICA,DK,MUNKSGAARD, COPENHAGEN, Bd. 52, 1998, Seiten 503-507, XP000857743 ISSN: 0904-213X
- R E DOLLE ET AL: "Improved Preparation of (3.beta.,5.alpha.,14.alpha)-3-Hydroxy-14-m eth ylcholest-7-en-15-one. Synthesis of Ergostenone and 20.alpha.-(Hydroxymethyl)pregnenone Analogues" JOURNAL OF ORGANIC CHEMISTRY,US,AMERICAN CHEMICAL SOCIETY. EASTON, Bd. 51, Nr. 21, 17. Oktober 1986 (1986-10-17), Seiten 4047-4053-4053, XP002111486 ISSN: 0022-3263
- J VAN DER EYCKEN ET AL: "24(R),25-Dihydroxycholesterol;An Attempt for Side Chain Stereocontrol via Iodolactonization" BULLETIN DES SOCIETES CHIMIQUES BELGES,GB,PERGAMON PRESS LTD. OXFORD, Bd. 95, Nr. 4, April 1986 (1986-04), Seiten 289-292-292, XP002111487
- RUAN B ET AL: "An alternative synthesis of 4,4-Dimethyl-5alpha-cholesta-8,14,24-trie n-3beta-ol, an intermediate in sterol biosynthesis and a reported activator of meiosis and of nuclear orphan receptor LXRalpha" BIOORGANIC & MEDICINAL CHEMISTRY LETTERS,GB,OXFORD, Bd. 8, Nr. 3, 3. Februar 1998 (1998-02-03), Seiten 233-236, XP004136854 ISSN: 0960-894X
- A. JARZEBSKI ET AL: "A facile approach to 20-isocholesterol from an androstane derivative" SYNTHETIC COMMUNICATIONS, Bd. 19, Nr. 1/2, 1989, Seiten 63-70, XP002139368

## Beschreibung

Die Erfindung betrifft Verfahren zur Herstellung von 4,4-Dimethyl-3β-hydroxypregna-8,14-dien-21-carbonsäureestern (**1**) und Zwischenprodukte im Verfahren 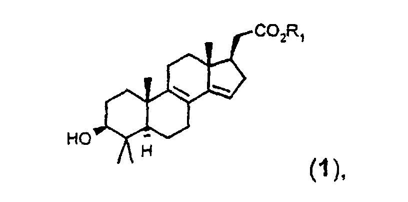 worin R¹ = Wasserstoff, verzweigtes oder unverzweigtes C₁ - C₆ Alkyl, Phenyl, Benzyl, ortho-, meta- oder para-Methylphenyl bedeuten,
und die Verwendung zur Herstellung von 4,4-Dimethyl-5α-cholesta-8,14,24-trien-3β-ol (**2**) (**FF-MAS**).

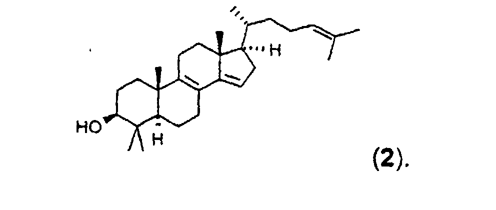

Untersuchungen von Byskov et. al. (*Nature* **1995**, *374*, 559) zeigen, daß 4,4-Dimethyl-5α-cholesta-8,14,24-trien-3β-ol , Formel **2,** im folgenden **FF-MAS** genannt, isoliert aus menschlicher Follikelflüssigkeit, eine die Meiose regulierende endogene Substanz ist, der interessante hormonelle Effekte zugeschrieben werden. Somit ist diese Substanz für pharmazeutische Anwendungen, beispielsweise zur Fertilitätsförderung, von Bedeutung.

Eine erste Synthese dieses Naturstoffes, welcher in der Biosynthese von Cholesterin aus Lanosterin durchlaufen wird, wurde von Dolle et al. (*J. Am. Chem. Soc*. **1989**, *111*, 278) beschrieben. Ausgehend von Ergosterin wird FF-MAS in einer 18stufigen aufwendigen Synthesesequenz erhalten. Große Teile der Synthese sind dem chemischen Teilabbau der Ergosterinseitenkette, dem anschließenden Aufbau der FF-MAS Seitenkette und der zur Erreichung dieses Zieles notwendigen Schutzgruppenchemie gewidmet.

Eine zweite Synthese von **FF-MAS** wurde von Schroepfer et al. ausgehend von Dehydrocholesterol in einer 13stufigen Synthese beschrieben (Bioorg. Med. Chem. Lett. **1997,** 8, 233). Auch in dieser Synthese muß zum Seitenkettenaufbau ein aufwendiger Schutz des Dien-Systems durchgeführt werden. Allein vier Schritte (Epoxydierung und Umlagerung zum Schutz; Reduktion und Eliminierung zur Regeneration des Dien-Systems) sind der Schutzgruppenstrategie geschuldet.

Eine dritte Synthese von **FF-MAS** wurde von Ruan et al. (Med. Chem. Letters **1998**, 233) entwickelt. Dabei wird ausgehend von Cholesteron in einer 15stufigen Synthese **FF-MAS** aufgebaut. Große Teile der Synthese sind dabei dem aufwendigen Aufbau des Doppelbindungssystems im Steroid und dem Aufbau der Seitenkette gewidmet.

Weitere Verfahren werden innerhalb der noch unveröffentlichten DE 198 17 520 und 198 23 677 beschrieben. Diese Synthesen gehen vom 3-Oxopregn-4-en-21-carbonsäureester aus. Zentrale Zwischenprodukte dieser Verfahren sind die unter der allgemeinen Formel 1 beschriebenen 4,4-Dimethyl-3β-hydroxypregna-8,14-dien-21-carbonsäureester.

Aufgabe dieser Erfindung sind neue Verfahren zur Synthese dieser zentralen Zwischenstufen. Ebenfalls Gegenstand dieser Erfindung sind die neuen, bisher nicht bekannten Zwischenprodukte, die im Rahmen der Synthesen durchlaufen werden und per se oder derivatisiert als Ausgangsmaterialien für die Synthese anderer Zielmoleküle verwendet werden können, beispielsweise zur Synthese von FF-MAS Analoga ( siehe WO 96/00235) und die Verwendung von Verbindungen zur Herstellung von 4,4-Dimethyl-5α-cholesta-8,14,24-trien-3β-ol.

Gelöst wird diese Aufgabe durch die Lehre der Patentansprüche.

Durch das erfindungsgemäße Verfahren werden weniger Zwischenstufen durchlaufen als in den bekannten Synthesen aus dem Stand der Technik, und die Anzahl der Reinigungsschritte sind deutlich niedriger.

### Erfindungsgemäßes Verfahren:

Gemäß **Schema 1** werden 4,4-Dimethyl-3β-hydroxypregna-8,14-dien-21-carbonsäureester der allgemeinen Formel **1** ausgehend von Androstendion (**3**) in einer 5stufigen Sequenz hergestellt.
Das als Ausgangsmaterial verwendete Adrostendion ist kommerziell erhältlich.

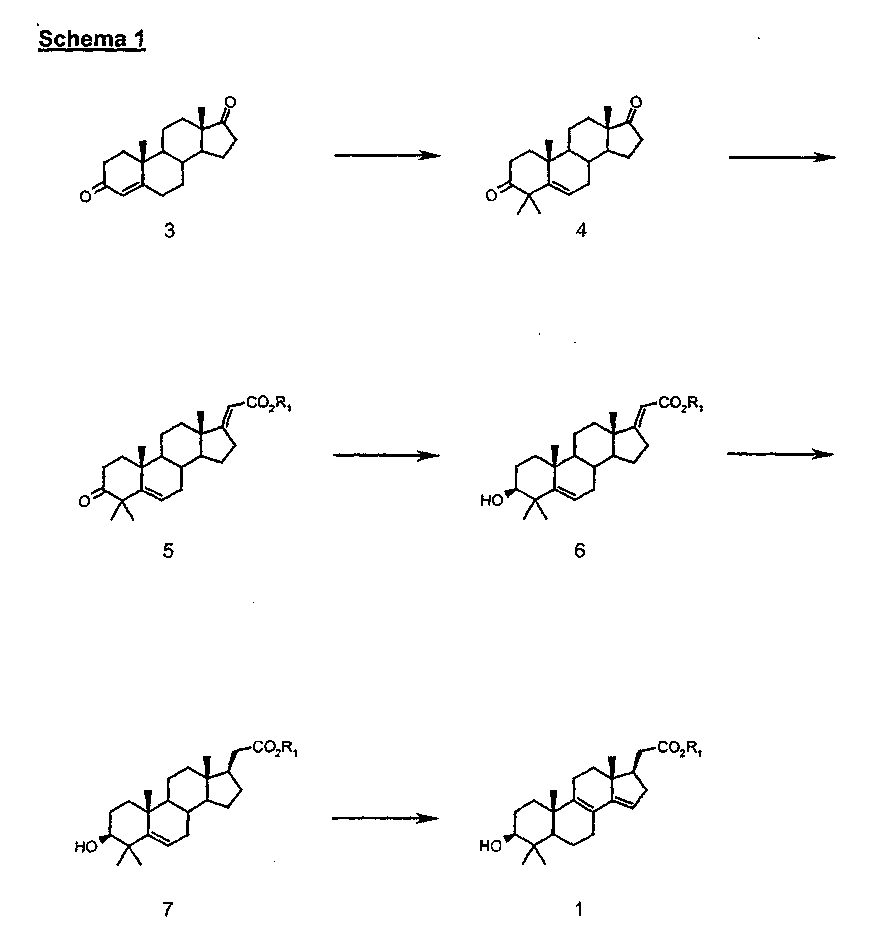

Die Umsetzung einer Verbindung der Formel **3** zu einer Verbindung der Formel **4** erfolgt nach an sich bekannten Verfahren (z.B. *Helv. Chim. Acta* **1980,** *63*, 1554; *J. Am. Chem. Soc*. **1954,** *76*, 2852). Beispielsweise wird eine Verbindung der Formel **3** in Gegenwart von Basen wie etwa den Alkalisalzen niederer Alkohole, bevorzugt aber Kaliumtertiärbutylat mit einem Alkylierungsmittel wie etwa Dimethylsulfat, Dimethylcarbonat oder auch Methyliodid in einem Lösemittel oder Lösemittelgemisch umgesetzt. Als Lösemittel können niedere, bevorzugt tertiäre Alkohole sowie Ether, beispielsweise Methyltertiärbutylether oder Tetrahydrofuran und deren Gemische verwendet werden. Bevorzugt ist die Verwendung von tertiär-Butanol bzw. eines Gemisches aus tertiär-Butanol und Tetrahydrofuran. Die Reaktion wird in einem Temperaturbereich von 0 °C bis 65 °C durchgeführt, bevorzugt aber im Temperaturbereich von 15 °C bis 50 °C.

Die Umsetzung einer Verbindung der Formel 4 zu einer Verbindung der Formel **5** erfolgt nach an sich bekannten Verfahren (z.B. Synth. Commun. **1977,** 7, 215; JOC **1988,** 3947; J. Prakt. Chem. **1990,** 367). Beispielsweise wird eine Verbindung der Formel **4** in Gegenwart von Basen wie etwa den Alkalisalzen niederer Alkohole, bevorzugt aber Natriumethylat, mit einem Trialkylphosphonoacetat wie etwa Triethylphosphonoacetat oder Trimethylphosphonoacetat in einem Lösemittel oder Lösemittelgemisch umgesetzt. Als Lösemittel können niedere, bevorzugt primäre Alkohole sowie Ether, beispielsweise Methyltertiärbutylether oder Tetrahydrofuran und deren Gemische verwendet werden. Bevorzugt ist die Verwendung von Ethanol. Die Reaktion wird in einem Temperaturbereich von 0 °C bis 100 °C durchgeführt, bevorzugt aber im Temperaturbereich von 20 °C bis 80 °C.

Ausgehend von einer Verbindung der Formel **4** ist eine Verbindung der Formel **5** auch via Kondensation mit Meldrumsäure oder Malonsäureestern, anschließender Verseifung und Decarboxylierung und Veresterung darstellbar.

Dem Fachmann ist geläufig, daß R¹ in Verbindungen der Formel **5** nach Standardmethoden variiert werden kann. Dies kann durch Verwendung anderer Alkohole im Veresterungsschritt geschehen, aber auch durch Umesterung eines schon vorhandenen Esters. R¹ kann also die Bedeutung von Wasserstoff, Methyl, Ethyl, Propyl, Isopropyl, Butyl und die entsprechenden Butylisomere, Pentyl und die entsprechenden Pentylisomere sowie Hexyl und die entsprechenden Hexylisomere, Phenyl, Benzyl, ortho-, meta- und para-Methylphenyl besitzen.

Die Umsetzung eines Ketons der Formel **5** in den entsprechenden 3-Alkohol der Formel **6** läßt sich mit einer Vielzahl von Reduktionsmitteln durchführen. Als Beispiele seien genannt: BH₃-Komplexe (z.B. mit tertiär-Butylamin oder Trimethylamin), Selectride, Natrium- und Lithiumborhydrid, gebremste Lithiumaluminiumhydride (z.B. LiAl(O^{t}Bu)₃H); auch sind Mikroorganismen wie z.B. Bäckerhefen oder Enzyme, beispielsweise 3β-Hydroxysteroiddehydrogenase, verwendbar.

Dem Fachmann ist bekannt, daß je nach verwendetem Reagenz verschiedene Lösemittel oder Lösemittelgemische und Reaktionstemperaturen zum Einsatz kommen. Bevorzugt werden hier jedoch Borhydride, wie etwa Natriumborhydrid in geeigneten Lösemitteln wie etwa niederen Alkoholen oder Gemische von Alkoholen mit anderen Lösemitteln, beispielsweise Dichlormethan , Tetrahydrofuran oder Wasser. Die Umsetzungen werden in einem Temperaturbereich von -20 °C bis 40 °C, bevorzugt jedoch im Bereich von -10 °C bis 10 °C durchgeführt.

Die Reduktion der 17-Doppelbindung in den Verbindungen der allgemeinen Formel **6** ist nach an sich bekannten Verfahren möglich. Dabei können zwei prinzipiell verschiedene Verfahren zum Einsatz kommen.

Ein geeignetes Reduktionsmittel ist hierbei in Anlehnung an literaturbekannte Umsetzungen (Synthesis 1996, 455) ein Gemisch aus Erdalkalimetallen in niederen Alkoholen. Beispielsweise wird eine Verbindung der allgemeinen Formel 6 in einem niederen Alkohol, bevorzugt Methanol, mit einem Erdalkalimetall, bevorzugt Magnesium, zur Reaktion gebracht. Die Reaktion wird in einem Temperaturbereich von 0 °C bis 80 °C durchgeführt, bevorzugt aber in einem Temperaturbereich von 20 °C bis 50 °C.

Als weiteres Reduktionsverfahren bietet sich in diesem Fall die katalytische Hydrierung an. Beispielsweise wird eine Verbindung der Formel **6** in Gegenwart eines geeigneten Katalysators wie etwa Edelmetalle oder deren Oxide, bevorzugt aber Platinoxid, hydriert. Als Lösemittel können nieder Alkohole, bevorzugt Ethanol, sowie Ether, beispielsweise Methyltertiärbutylether oder Tetrahydrofuran oder deren Gemische verwendet werden. Bevorzugt ist die Verwendung von Tetrahydrofuran. Überraschenderweise wird dabei die 5,6-Doppelbindung nicht hydriert.

Von Vorteil hat sich die Zugabe von katalytischen Mengen Säure wie etwa Schwefelsäure, Phosphorsäure oder Zitronensäure erwiesen. Bevorzugt ist die Verwendung von Phosphorsäure. Die Umsetzung wird in einem Temperaturbereich von 10 °C bis 100 °C durchgeführt; sie kann sowohl unter Normaldruck wie auch unter erhöhtem Druck durchgeführt werden. Bevorzugt ist hierbei die Umsetzung im Temperaturbereich von 20 °C bis 50 °C und unter Normaldruck.

Die Einführung der 7,8-Doppelbindung und die isomerisierung der Doppelbindungen zu dem in der Zielverbindung etablierten Doppelbindungssystem kann durch Bromierung/Dehydrobromierung/lsomerisierung (es geht auch der Weg über das entsprechende Chlorid und Dehydrochlorierung) in einem Eintopfverfahren erreicht werden.

Zunächst wird allylisch zur 5,6-Doppelbindung in Position 7 bromiert und anschließend durch thermische Eliminierung von Bromwasserstoff das 5,7-Doppelbindungssystem erhalten, welches durch saure Isomerisierung in das gewünschte Doppelbindungssystem übergeht. Die Zugabe von Säure ist nicht nötig; der zwischenzeitlich gebildete Bromwasserstoff übernimmt diese Aufgabe zufriedenstellend.

Die Bromierung erfolgt nach an sich bekannten Verfahren. Beispielsweise kann N-Bromsuccinimid oder N,N-Dibromdimethylhydantoin in einem geeigneten Lösemittel, wie etwa Benzol, niederen Alkanen oder auch halogenierten Kohlenwasserstoffen wie etwa Tetrachlorkohlenstoff verwendet werden. Es können aber auch andere als die bisher genannten Lösemittel eingesetzt werden, beispielsweise Ameisensäuremethylester (z.B.: Angew. Chem. **1980**, 92, 471).

Bevorzugt ist die Verwendung von Heptan als Lösemittel. Die Reaktion wird in einem Temperaturbereich von 30 °C bis 130 °C durchgeführt, bevorzugt aber im Temperaturbereich von 60 °C bis 100 °C.

### Beispiele

### a) 4,4-Dimethylandrostendion (4):

Zu 500 g Androstendion in 5 l tertiär-Butanol werden 411 g Kaliumtertiärbutylat bei RT zugegeben. Dann werden 229 ml Methyljodid zugetropft, und das Gemisch wird 1 h nachgerührt. Zur Aufarbeitung werden 400 ml 1 M H₂SO₄ und anschließend 2 l Wasser zugefügt. Der Niederschlag wird abfiltriert und aus Ethanol umkristallisiert. Es werden 413 g 4,4-Dimethylandrostendion erhalten.
¹H-NMR (CDCl₃): δ = 0.89 und 0.90 (2 s, 3H, 18- und 19-H₃), 1.06-2.66 (m, 17H, Androstendion), 1.25 [s, 6H, 4-(CH₃)₂], 5.58-5.61 (m, 1H, 6-H).

| | | | |
|---|---|---|---|
| Smp.: 165-167°C, Verbrennungsanalyse: | Ber. | C 80.21 | H 9.62 |
| | Gef. | C79.96 | H 9.61 |

### b) (20E)-4,4-Dimethyl-3-oxopregna-5,17-dien-21-carbonsäureethylester (5):

Zu 310 g 4,4-Dimethylandrostendion in 818 ml Ethanol werden 837 ml 20 %ige Natriumethylat-Lösung und 387 ml Triethylphosphonoacetat zugegeben. Das Gemisch wird 5 h unter Rückfluß gekocht, anschließend die Reaktion durch Zugabe von 1.6 l Wasser beendet. Der Niederschlag wird abfiltriert, nachgewaschen und getrocknet. Es werden 369 g (20E)-4,4-Dimethyl-3-oxopregna-5,17-dien-21-säureethylester erhalten.
¹H-NMR (CDCl₃): δ = 0.85 und 0.88 (2 s, 3H, 18- und 19-H₃), 1.02-2.91 (m, 17 H, Androstendion), 1.26 [s, 6H, 4-(CH₃)₂], 1.29 (t, 3H, *J =* 7.0, CO₂CH₂C*H*₃), 4.15 (q, 2H, *J* = 7.1, CO₂C*H*₂CH₃), 5.55-5.58 (m, 2H, 6-H und 20-H).
Smp.: 136-138°C.

### c) (20E)-4,4-Dimethyl-3β-hydroxy-pregna-5,17-dien-21-carbonsäureethylester (6):

200 g der in Stufe b) beschriebenen Verbindung werden in 2 l Ethanol vorgelegt und mit 20 g Natriumborhydrid in 0.4 l Wasser bei 0 °C versetzt. Anschließend wird 11 h gerührt. Zum Reaktionsgemisch wird eine Lösung von 328 g Citronensäure in 2.8 l Wasser gegeben und nach 1 h der Feststoff abgetrennt. Der Rückstand wird mehrfach mit Wasser gewaschen und i. V. getrocknet. Es resultieren 190 g (20E)-4,4-Dimethyl-3β-hydroxy-pregna-5,17-dien-21-säureethylester, welcher ohne Reinigung weiterverwendet wird.
¹H-NMR (CDCl₃): δ = 0.83, 1.08, 1.10 und 1.15 [4 s, 3H, 4-(CH₃)₂, 18- und 19-H₃], 0.91-2.90 (m, 17 H, Androstendion), 1.28 (t, 3H, *J* = 7.1, CO₂CH₂C*H*₃), 3.24 (dd á 1H, *J* = 10.2, 5.5, 3-H), 4.15 (q, 2H, *J* = 7.1, CO₂C*H*₂CH₃), 5.53-5.59 (m, 2H, 6-H und 20-H).

| | | | |
|---|---|---|---|
| Smp.: 171-173°C, Verbrennungsanalyse: | Ber. | C 77.68 | H 9.91 |
| | Gef. | C 77.75 | H |

### d) 4,4-Dimethyl-3β-hydroxy-pregn-5-en-21-carbonsäureethylester (7) (durch Hydrierung):

200 g (20E)-4,4-Dimethyl-3β-hydroxy-pregna-5,17-dien-21-carbonsäureethylester werden in 1.2 l THF gelöst und mit 0.4 ml 85 %iger Phosphorsäure und 4 g Platinoxid versetzt. Dann wird das Reaktionsgefäß mit Wasserstoff (1 bar) begast. Nach Beendigung der Wasserstoffaufnahme wird vom Katalysator filtriert und das Lösungsmittel abdestilliert. Es resultieren 210 g 4,4-Dimethyl-3β-hydroxy-pregn-5-en-21-säureethylester, welcher ohne Reinigung weiterverwendet wird.

### 4,4-Dimethyl-3β-hydroxy-pregn-5-en-21-carbonsäureethylester (7) (durch Magnesiumreduktion):

5.0 g (20E)-4,4-Dimethyl-3β-hydroxy-pregna-5,17-dien-21-säureethylester werden bei RT in 100 ml Methanol gelöst und mit 0.5 ml Essigsäure versetzt. Dann werden portionsweise Magnesiumspäne zum Gemisch gegeben. Nach 2.5 h wird mit 25 ml Essigsäure angesäuert und anschließend mit 200 ml Wasser versetzt. Der Niederschlag wird abfiltriert, mit Wasser nachgewaschen und getrocknet. Es resultieren 4.7 g 4,4-Dimethyl-3β-hydroxy-pregn-5-en-21-säureethylester, welcher ohne Reinigung weiterverwendet wird.
¹H-NMR (CDCl₃): δ = 0.61, 1.08, 1.10 und 1.15 [4 s, 3H, 4-(CH₃)₂, 18- und 19-H₃], 0.90-2.42 (m, 18 H, Androstendion), 1.25 (t, 3H, *J* = 7.1, CO₂CH₂C*H*₃), 3.22-3.26 (m, 1H, 3-H), 4.11 (q, 2H, *J* = 7.1, CO₂C*H*₂CH₃), 5.55-5.58 (m, 1H, 6-H).

| | | | |
|---|---|---|---|
| Smp.: 127-129°C, Verbrennungsanalyse: | Ber. | C 77.27 | H 10.38 |
| | Gef. | C 77.00 | H 10.20. |

### e) 4,4-Dimethyl-3β-hydroxypregna-8,14-dien-21-carbonsäureethylester (1):

100 g der in Stufe d) beschriebenen Verbindung werden mit 48 g 1,3-Dibrom-5,5-dimethylhydantoin in 2.5 l n-Heptan 20 h refluxiert. Nach Abkühlung wird das Gemisch mit Essigester extrahiert, die organische Phase mehrfach mit Wasser gewaschen und eingeengt. Es werden 50 g 4,4-Dimethyl-3β-hydroxypregna-8,14-dien-21-säureethylester erhalten.
¹H-NMR (CDCl₃): δ = 0.75, 0,83, 1.02 und 1.03 [4 s, 3H, 4-(CH₃)₂, 18- und 19-H₃], 0.62-2.59 (m, 17 H, Androstendion), 1.26 (t, 3H, *J* = 7.1, CO₂CH₂C*H*₃), 3.25 (dd, 1H, *J* = 11.4, 4.8, 3-H), 4.13 (q, 2H, *J* = 7.1, CO₂C*H*₂CH₃), 5.35 (br. s, 1H, 15-H).
MS (ber. 386.58): .M+-Peak bei 387.

Weiterverarbeitung zum 4,4-Dimethyl-5α-cholesta-8,14,24-trien-3β-ol (**2**) **(FF-MAS):**

### f) Beginnend beim 4,4-Dimethyl-3β-hydroxypregna-8,14-dien-21 -säuremethylester weiter zum

### g) 4,4-Dimethyl-3β-[[dimethyl(1,1-dimethylethyl)silyl]oxy]pregna-8,14-dien-21-säuremethylester,

92 g 4,4-Dimethyl-3β-hydroxypregna-8,14-dien-21-säuremethylester werden mit 0,75 Litern N,N-Dimethylformamid, 51 g tertiär-Butyldimethylsilylchlorid und 27,8 g Imidazol 18 Stunden bei 70 °C gerührt. Nach Abkühlung wird auf 10 Liter einer eiskalten 0,5 molaren wässrigen Salzsäure gegossen und filtriert. Der Filterkuchen wird in Ethylacetat aufgenommen, mit 1 normaler Natronlauge neutral gewaschen, über Natriumsulfat getrocknet, filtriert und eingeengt. Es werden
124,8 g 4,4-Dimethyl-3β-[[dimethyl(1,1-dimethylethyl)silyl]oxy]pregna-8,14-dien-21-säuremethylester erhalten, welcher ohne Reinigung weiterverwendet wird.

### h) 4,4-Dimethyl-3β-[[dimethyl(1,1-dimethylethyl)silyl]oxy]-5β-cholesta-8,14,24-trien-21-säuremethylester

Zu einer Lösung von 1,04 mol Lithiumdiisopropylamid, hergestellt aus 652 ml einer 1,6 molaren Lösung von n-Butyllithium in Hexan und 174 ml Diisopropylamin in 320 ml Tetrahydrofuran werden bei-20 °C 123,5 g der in Stufe b) beschriebenen Verbindung, gelöst in 2,0 Litern Tetrahydrofuran, zugetropft. Nach 40 Minuten Rühren bei 0 °C wird auf -10 °C gekühlt und es werden 270 g 5-lodo-2-methyl-2-penten zugetropft. Nach 3stündigem Rühren bei 0 °C wird der Ansatz zwischen Ethylacetat und gesättigter Ammoniumchloridlösung verteilt. Nach Waschen der organischen Phase mit Wasser und gesättigter Kochsalzlösung, Trocknung über Natriumsulfat und Filtration wird eingeengt und grob über Kieselgel mit einem Gemisch aus n-Hexan und Ethylacetat filtriert. Es werden 113 g (0,2 mol) 4,4-Dimethyl-3β-[[dimethyl(1,1-dimethylethyl)-silyl]oxy]-5α-cholesta-8,14,24-trien-21-säuremethylester erhalten, welcher ohne Reinigung weiterverwendet wird.

### i) 4,4-Dimethyl-3β-[[dimethyl(1,1-dimethylethyl)silyl]oxy]-5α-cholesta-8,14,24-trien-21-ol

Zu 15,04 g Lithiumaluminiumhydrid, suspendiert in 0,7 Liter Tetrahydrofuran werden bei 0 °C 112,5 g der in Stufe c) beschriebenen Verbindung, gelöst in 0,7 Liter Tetrahydrofuran, getropft. Nach 3 Stunden Rühren bei Raumtemperatur wird unter Eiskühlung mit 60 ml gesättigter Ammoniumchloridlösung versetzt. Nach 20 Minuten Rühren wird mit Natriumsulfat versetzt und nach weiteren 10 Minuten abgesaugt. Der Eindampfrückstand wird über eine kurze Säule mit Dichlormethan als Lösemittel filtriert. Nach Einengen des Eluates werden 103,2 g 4,4-Dimethyl-3β-[[dimethyl(1,1-dimethylethyl)silyl]oxy]-5αcholesta-8,14,24-trien-21-ol erhalten, welches ohne weitere Reinigung weiterverwendet wird.

### j) 4,4-Dimethyl-3β-[[dimethyl(1,1-dimethylethyl)silyl]oxy]-5α-cholesta-8,14,24-trien-21-ol-methansulfonat

Bei 0 °C werden zu einer Lösung von 102,3 g der in Stufe d) beschriebenen Verbindung in einem Gemisch aus 440 ml Dichlormethan und 84 ml Triethylamin 21,8 ml Methansulfonsäurechlorid getropft. Nach 3 Stunden bei Raumtemperatur wird zwischen Wasser und Dichlormethan verteilt. Nach Waschen der organischen Phase mit Natriumhydrogencarbonatlösung, gesättigter Kochsalzlösung, Trocknung über Natriumsulfat, Filtration und Einengen wird an Kieselgel mit einem Gemisch aus Hexan und Ethylacetat chromatographiert. Es werden 78,2 g 4,4-Dimethyl-3β-[[dimethyl(1,1-dimethylethyl)silyl]oxy]-5α-cholesta-8,14,24-trien-21-ol-methansulfonat erhalten.

### k) 4,4-Dimethyl-3β-[[dimethyl(1,1-dimethylethyl)silyl]oxy]-5α-cholesta-8,14,24-trien

77,2 g der in Stufe e) beschriebenen Verbindung werden nach der in Stufe d) beschriebenen Methode umgesetzt. Nach Filtration des Rohproduktes über Kieselgel mit einem Gemisch aus n-Hexan und Ethylacetat werden 63 g 4,4-Dimethyl-3β-[[dimethyl(1,1-dimethylethyl)silyl]oxy]-5α-cholesta-8,14,24-trien erhalten.

### l) 4,4-Dimethyl-5α-cholesta-8,14,24-trien-3β-ol

2 g der in Stufe f) beschriebenen Verbindung werden in einem Gemisch aus 5 ml 6 normaler Salzsäure, 10 ml Ethanol und 30 ml Tetrahydrofuran 24 Stunden bei Raumtemperatur gerührt. Anschließend wird zwischen Ethylacetat und Wasser verteilt. Nach Waschen der organischen Phase mit 1 normaler Natronlauge, Wasser und gesättigter Kochsalzlösung, Trocknung über Natriumsulfat und Filtration wird der Eindampfrückstand an Kieselgel mit einem Gemisch aus n-Hexan und Ethylacetat chromatographiert.
Es werden 1,45 g 4,4-Dimethyl-5α-cholesta-8,14,24-trien-3β-ol erhalten.

Die NMR-Daten stimmen mit denen der Literatur überein (*J. Am. Chem. Soc. 111*, **1989**, 278)

## Patentansprüche

1. Verfahren zur Herstellung von 4,4-Dimethyl-3β-hydroxypregna-8,14-dien-21-carbonsäureestern der allgemeinen Formel **1** 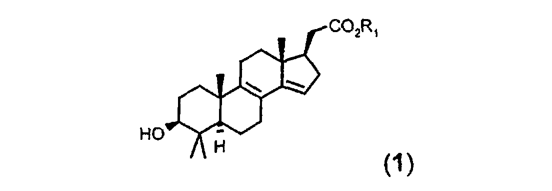 worin R¹ = Wasserstoff, verzweigtes oder unverzweigtes C₁ - C₆ Alkyl, Phenyl, Benzyl, ortho-, meta- oder para-Methylphenyl bedeuten,
aus Androstendion **3** 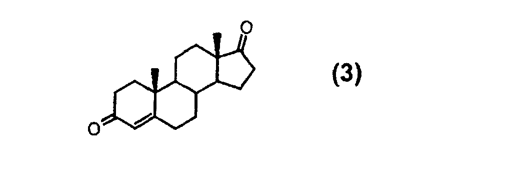
a.) durch Dimethylierung ins 4,4-Dimethylandrostendion der Formel **4** 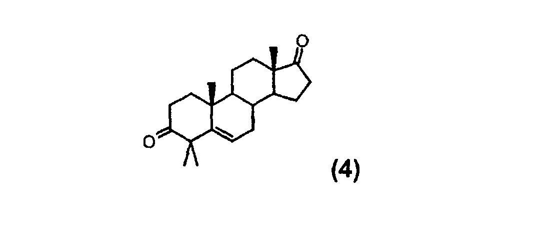
b.) durch Alkylierung in die 4,4-Dimethyl-3-oxopregna-5,17-dien-21-carbonsäureester der allgemeinen Formel **5** 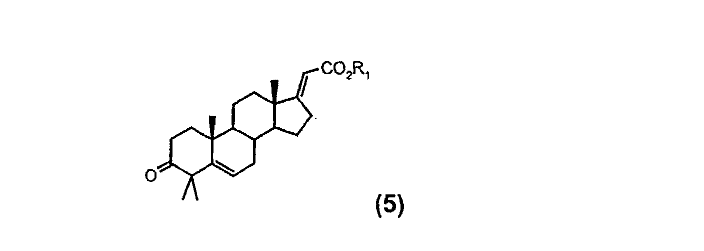 worin
R¹ = Wasserstoff, verzweigtes oder unverzweigtes C₁ - C₆ Alkyl, Phenyl, Benzyl, ortho-, meta- oder para-Methylphenyl bedeuten,
c.) durch Reduktion in die 4,4-Dimethyl-3β-hydroxy-pregna-5,17-dien-21-carbonsäureester der allgemeinen Formel **6** 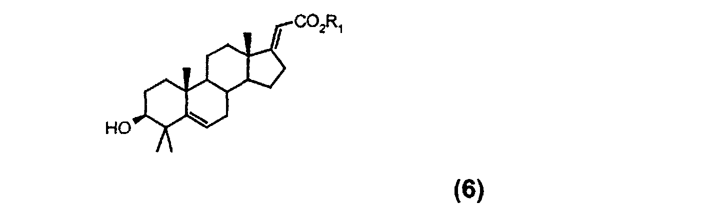 worin
R¹ = Wasserstoff, verzweigtes oder unverzweigtes C₁ - C₆ Alkyl, Phenyl, Benzyl, ortho-, meta- oder para-Methylphenyl bedeuten,
d.) durch Reduktion der 17-Doppelbindung in die 4,4-Dimethyl-3β-hydroxypregn-5-en-21-carbonsäureester der allgemeinen Formel **7** 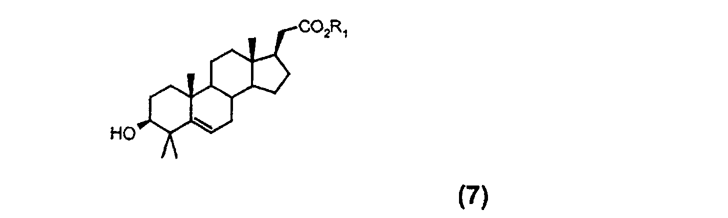
worin
R¹ = Wasserstoff, verzweigtes oder unverzweigtes C₁ - C₆ Alkyl, Phenyl, Benzyl, ortho-, meta- oder para-Methylphenyl bedeuten,
und durch anschließende Halogenierung, Dehydrohalogenierung und Isomerisierung und Überführung in die 4,4-Dimethyl-3β-hydroxypregna-8,14-dien-21-carbonsäureester der allgemeinen Formel (**1**).

2. 4,4-Dimethyl-3-oxopregna-5,17-dien-21-carbonsäureester der allgemeinen Formel **5** 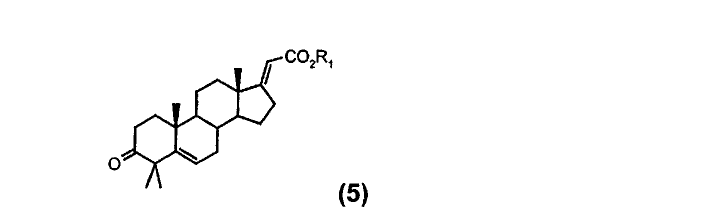 worin
R¹ = Wasserstoff, verzweigtes oder unverzweigtes C₁ - C₆ Alkyl, Phenyl, Benzyl, ortho-, meta- oder para-Methylphenyl bedeuten.

3. 4,4-Dimethyl-3β-hydroxy-pregna-5,17-dien-21-carbonsäureester der allgemeinen Formel **6** 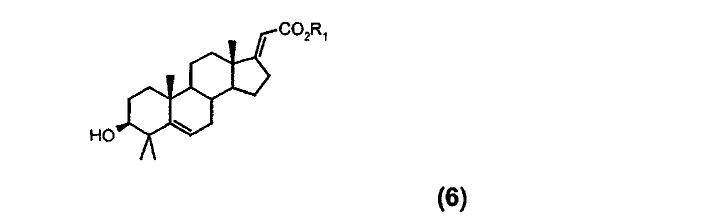 worin
R¹ = Wasserstoff, verzweigtes oder unverzweigtes C₁ - C₆ Alkyl, Phenyl, Benzyl, ortho-, meta- oder para-Methylphenyl bedeuten,

4. Verwendung von (**1**) zur Herstellung von FF-MAS.

5. 4,4-Dimethyl-3β-hydroxy-pregn-5-en-21-carbonsäureester der allgemeinen Formel **7** 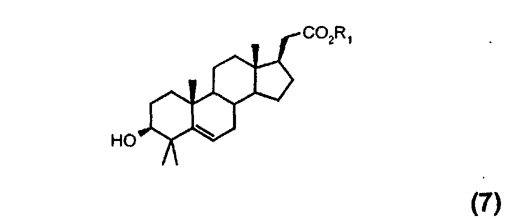 worin
R¹ = Wasserstoff, verzweigtes oder unverzweigtes C₁ - C₆ Alkyl, Phenyl, Benzyl, ortho-, meta- oder para-Methylphenyl bedeuten,

## Claims

1. Process for preparing 4,4-dimethyl-3β-hydroxypregna-8,14-diene-21-carboxylic esters of the general formula **1** 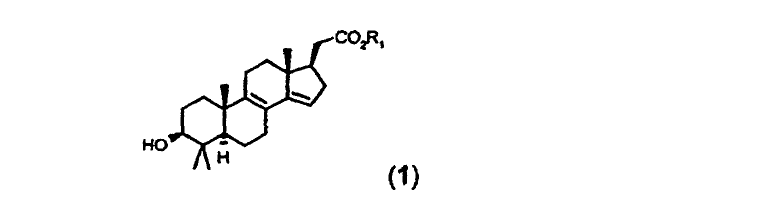 where R¹ = hydrogen, branched or unbranched C₁-C₆ alkyl, phenyl, benzyl, ortho-, meta- or para-methylphenyl, from androstenedione **3** 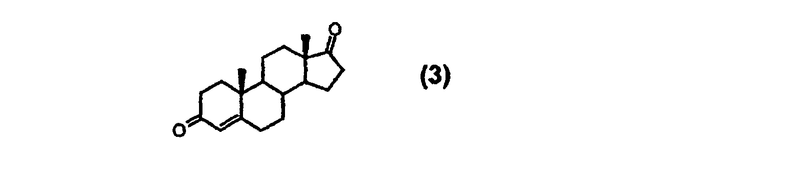
a. by dimethylation to give the 4,4-dimethylandrostenedione of the formula **4** 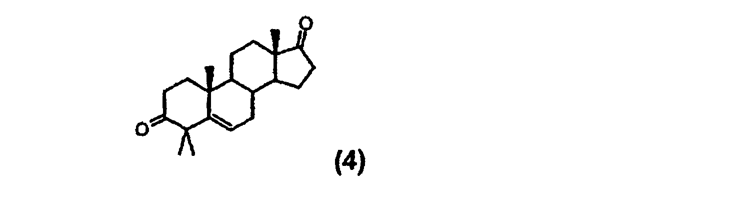
b. by alkylation to give the 4,4-dimethyl-3-oxopregna-5,17-diene-21-carboxylic ester of the general formula **5** 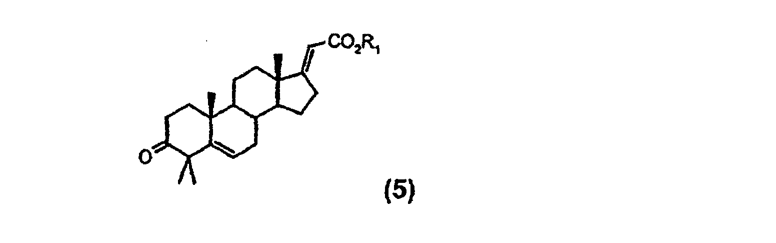 where
R¹ = hydrogen, branched or unbranched C₁-C₆ alkyl, phenyl, benzyl, ortho-, meta- or para-methylphenyl,
c. by reduction to give the 4,4-dimethyl-3β-hydroxypregna-5,17-diene-21-carboxylic ester of the general formula **6** 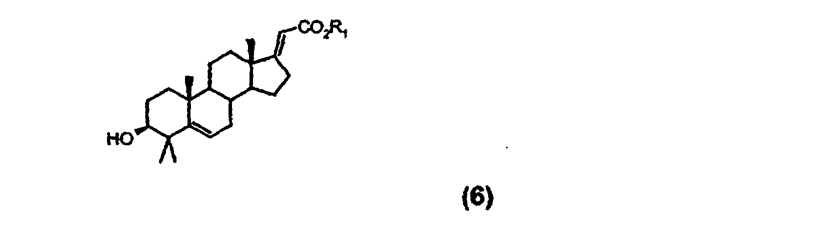 where
R¹ = hydrogen, branched or unbranched C₁-C₆ alkyl, phenyl, benzyl, ortho-, meta- or para-methylphenyl,
d. by reduction of the 17-double bond to give the 4,4-dimethyl-3β-hydroxypregn-5-ene-21-carboxylic ester of the general formula **7** 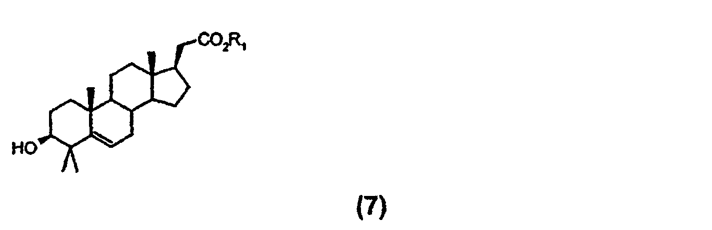 where
R¹ = hydrogen, branched or unbranched C₁-C₆ alkyl, phenyl, benzyl, ortho-, meta- or para-methylphenyl,
and by subsequent halogenation, dehydrohalogenation and isomerization and conversion to the 4,4-dimethyl-3β-hydroxypregna-8,14-diene-21-carboxylic esters of the general formula **(1)**.

2. 4,4-Dimethyl-3-oxopregna-5,17-diene-21-carboxylic esters of the general formula **5** 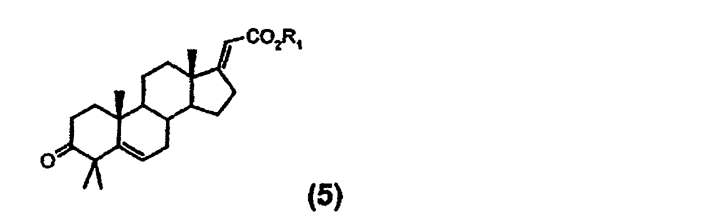 where
R¹ = hydrogen, branched or unbranched C₁-C₆ alkyl, phenyl, benzyl, ortho-, meta- or para-methylphenyl.

3. 4,4-Dimethyl-3β-hydroxypregna-5,17-diene-21-carboxylic esters of the general formula **6** 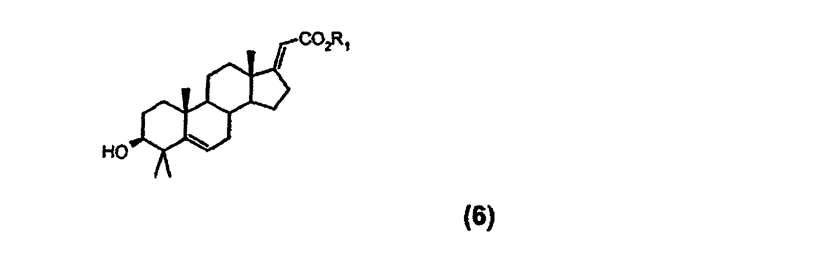 where
R¹ = hydrogen, branched or unbranched C₁-C₆ alkyl, phenyl, benzyl, ortho-, meta- or para-methylphenyl.

4. Use of (**1**) for preparing FF-MAS.

5. 4,4-Dimethyl-3β-hydroxypregn-5-ene-21-carboxylic esters of the general formula **7** 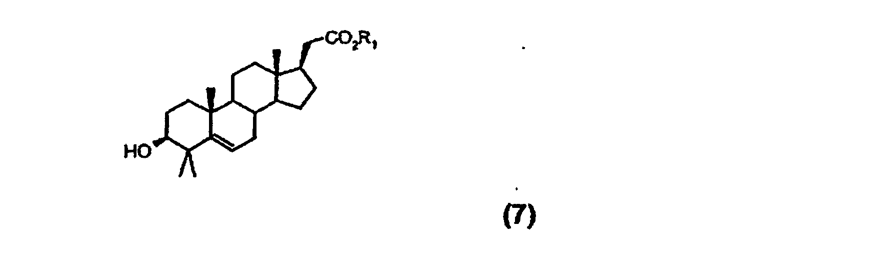 where
R¹ = hydrogen, branched or unbranched C₁-C₆ alkyl, phenyl, benzyl, ortho-, meta- or para-methylphenyl.

## Revendications

1. Procédé de préparation d'esters 4,4-diméthyl-3β-hydroxyprégna-8,14-diène-21-carboxyliques de formule générale 1 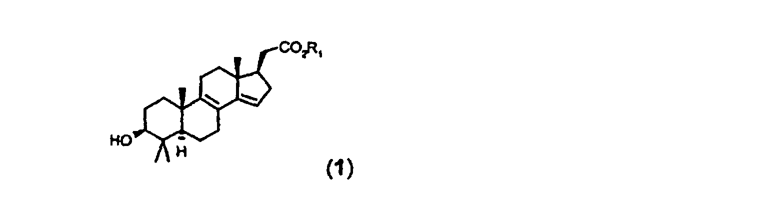 dans laquelle R¹ représente un hydrogène, un alkyle en C₁-C₆ ramifié ou non ramifié, un phényle, un benzyle, un ortho-, un méta- ou un para-méthylphényle, à partir de l'androstènedione 3 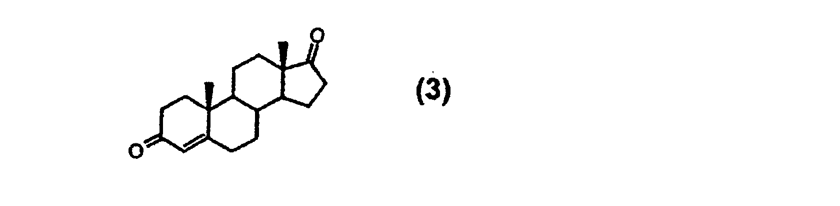
a) par diméthylation pour donner lieu à la 4,4-diméthylandrostènedione de formule 4 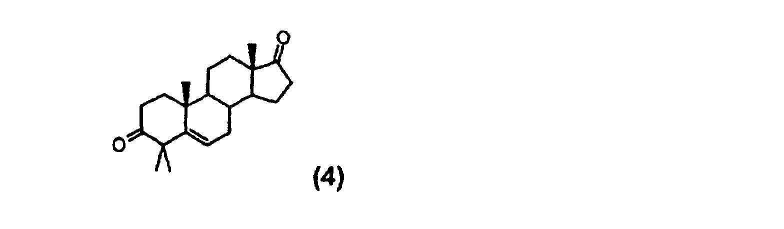
b) par alkylation pour donner lieu aux esters 4,4-diméthyl-3-oxoprégna-5,17-diène-21-carboxyliques de formule générale 5 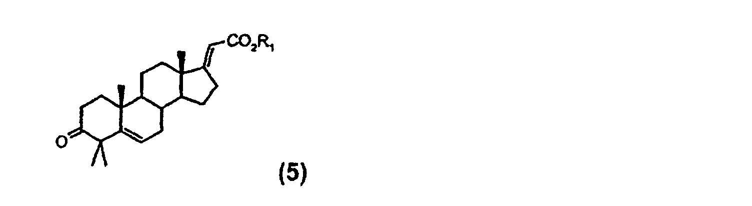 dans laquelle
R¹ représente un hydrogène, un alkyle en C₁-C₆ ramifié ou non ramifié, un phényle, un benzyle, un ortho-, un méta- ou un para-méthylphényle,
c) par réduction pour donner lieu aux esters 4,4-diméthyl-3β-hydroxyprégna-5,17-diène-21-carboxyliques de formule générale 6 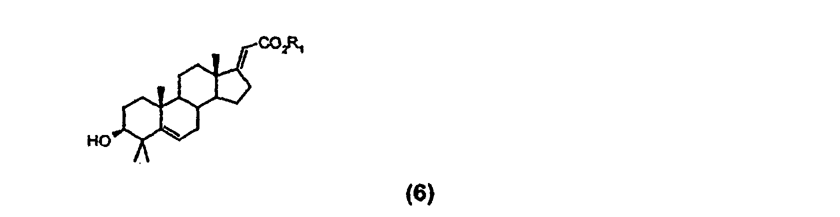 dans laquelle
R¹ représente un hydrogène, un alkyle en C₁-C₆ ramifié ou non ramifié, un phényle, un benzyle, un ortho-, un méta- ou un para-méthylphényle,
d) par réduction de la double liaison en 17 pour donner lieu aux esters 4,4-diméthyl-3β-hydroxyprégn-5-ène-21-carboxyliques de formule générale 7 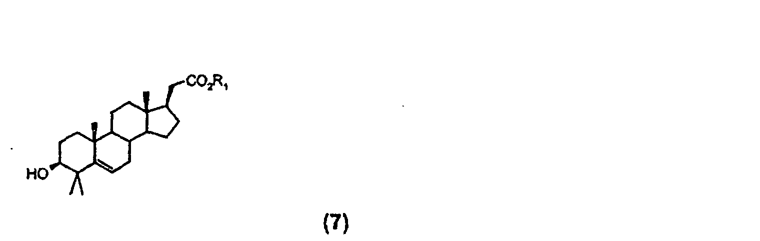 dans laquelle
R¹ représente un hydrogène, un alkyle en C₁-C₆ ramifié ou non ramifié, un phényle, un benzyle, un ortho-, un méta- ou un para-méthylphényle,
et par halogénation, déhydrohalogénation et isomérisation subséquentes et transformation en les esters 4,4-diméthyl-3β-hydroxyprégna-8,14-diène-21-carboxyliques de formule générale (1).

2. Esters 4,4-diméthyl-3-oxoprégna-5,17-diène-21-carboxyliques de formule générale 5 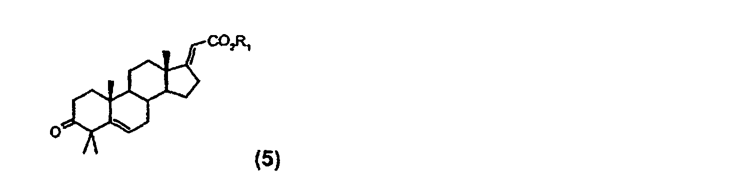 dans laquelle
R¹ représente un hydrogène, un alkyle en C₁-C₆ ramifié ou non ramifié, un phényle, un benzyle, un ortho-, un méta- ou un para-méthylphényle.

3. Esters 4,4-diméthyl-3β-hydroxyprégna-5,17-diène-21-carboxyliques de formule générale 6 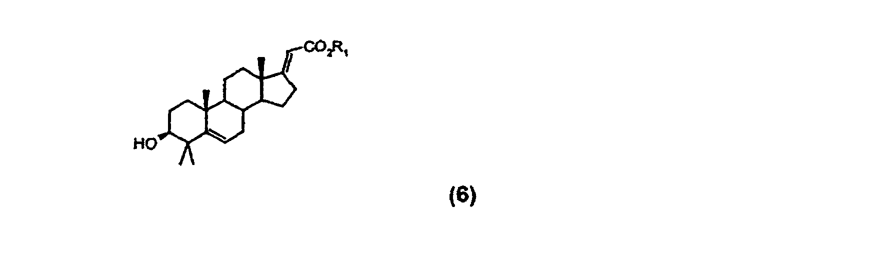 dans laquelle
R¹ représente un hydrogène, un alkyle en C₁-C₆ ramifié ou non ramifié, un phényle, un benzyle, un ortho-, un méta- ou un para-méthylphényle.

4. Utilisation de (1) pour la préparation de FF-MAS.

5. Esters 4,4-diméthyl-3β-hydroxyprégn-5-ène-21-carboxyliques de formule générale 7 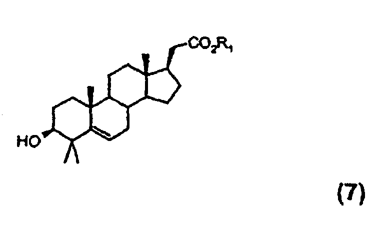 dans laquelle
R¹ représente un hydrogène, un alkyle en C₁-C₆ ramifié ou non ramifié, un phényle, un benzyle, un ortho-, un méta- ou un para-méthylphényle.
